# EUROPEAN PATENT SPECIFICATION

(11) **EP 2 198 783 B1**
(45) Date of publication and mention of the grant of the patent: **12.10.2016**
(21) Application number: 08172473.4
(22) Date of filing: 19.12.2008
(51) Int. Cl.: A61B 6/14, A61B 6/00

(54) **Apparatus and method for digital X-ray scanning**
Vorrichtung und Verfahren zur digitalen Röntgenabtastung
Appareil et procédé de balayage numérique à rayons X

(43) Date of publication of application: 23.06.2010
(73) Proprietor: CEFLA S.C., 40026 Imola (IT)
(72) Inventor: Rotondo, Giuseppe, 20090 Pantigliate - Milano (IT); Frosio, Iuri, 24124 Bergamo (IT); Rinaldi, Gerardo, 20124 Milano (IT); Venturino, Gianfranco, 20159 Milano (IT); Borghese, Nunzio Alberto, 20129 Milano (IT)
(74) Representative: Herrmann, Franz

(56) References cited:
- WO-A-01/60257
- US-A1- 2002 085 673
- US-A1- 2004 190 678

## Description

The invention relates to a method for digital X-ray scanning for dental cephalography, the method comprising the steps of:
- positioning a patient with respect to a radiation source and a detector using a patient support system;
- generating X-ray radiation by the radiation source;
- performing a scan of the patient by performing a roto-translation of a rotary support supporting the radiation source and a primary collimator disposed between the radiation source and the patient support system;
- moving a second collimator in synchronization with the rotary support;
- detecting the X-ray radiation by means of the detector;
- performing a readout of the detector for generating a radiographic image of the patient.

The invention further relates to an apparatus arranged for performing the method.

Such a method and such an apparatus are known from US 2004/190678 A1.

US 67 31 717 B2 discloses a method in which a fan shaped beam is produced by means of an X-ray source and a primary collimator that can be pivoted around a rotational center so that the head of a patient is scanned by the X-ray beam. In the known method, a second collimator is placed in front of the head of the patient. Behind the head of the patient a linear detector is disposed. The second collimator and the detector perform a linear movement such that the X-ray beam always impinges on the linear detector during the scanning procedure.

A first problem is particularly relevant for radiographic applications requiring a long Source Image Distance (= SID) such as Cephalography with a typical SID in the range of 1,5 m. The problem is that a dual collimator system is required to ensure an accurate collimation of the fan beam on the detector active area. In practice, a primary collimator in the vicinity of the source for rough collimation and a secondary collimator in proximity to the patient for fine collimation are provided. The independent motion of both collimators usually requires separate motor drives with accurate synchronization during the scanning movements. Furthermore, if the secondary collimator is linearly translated, the effective secondary aperture is slightly varying during the scanning process resulting in a reduction of the effective aperture at increasing angle of deflection.

A second problem is that the flexibility of operation and the economics of the scanning system depend strongly on the way the fan beam is generated and the capabilities of the cinematic system. For example, it is preferable to have a scanning system which is flexible in setting the geometry of projections from predefined virtual centers and SIDs.

A third problem is that, depending on the radiographic application, the body parts of the patient vary considerably regarding the absorption of the X-ray radiation during the scanning process. For instance, the irradiation of soft tissue may result in strong detector signals whereas hard or bony tissue result in weak detector signals so that the signal-to-noise ratio assumes inappropriate low values. The varying absorption of the tissue may also result in underexposed or overexposed radiographs.

US 70 16 461 B2 and US 65 84 171 B2 describe methods for reading out X-ray detectors during a scanning process, in particular a time-delay integration synchronized with the scanning motion.

US 71 03 141 B2 discloses various methods for modulating the intensity of the fan shaped X-ray beam during a scanning process, among which control of tube output voltage and of current and control of scanning speed.

BLANZ, V. and VETTER, T.: A Morphable Model for the Synthesis of 3D Faces, SIGGRAPH 99 Conference Proceedings, pp. 187-194 describes various methods for three-dimensional face modeling.

FROSIO, I.; LUCCHESE, M.; LISSANDRELLO, F.; BORGHESE, N. A.: Automatic Contrast Enhancement in Digital Radiography, Proc. MIC 2008, Dresden (Germany), October 20-26 2008 relates to methods for automatic image processing in digital radiography.

VELASCO, F. A.; MARROQUIN, J. L.: Growing Snakes: Active Contours for Complex Topologies, Pattern Recognition, Vol. 36 (2003), pp. 475 - 482 describes a so called snake algorithm for pattern recognition.

Proceeding from this related art, the present invention seeks to provide a method and an apparatus which avoids the drawbacks mentioned above by simplifying the geometry of the imaging.

This object is achieved by a method having the features of claim 1 and an apparatus according to claim 16 . Advantageous embodiments and refinements are specified in claims dependent thereon.

According to the method, the secondary collimator is mounted on the rotary support together with the radiation source and primary collimator. For scanning the patient, the secondary collimator is further moved together with the radiation source and the primary collimator by moving the rotary support. Thus, the secondary collimator remains adjusted to the primary collimator and the X-ray source independent of the position of the rotary support. In addition, the aperture of the secondary collimator remains constant during the scanning process. Furthermore, there is no need for an additional drive for moving the secondary collimator in synchronization with the motion of the primary collimator, so that the additional drive for the secondary collimator can be saved.

The roto-translation during the scanning process can be composed of a rotational motion around a rotation axis and a displacement of the rotation axis comprising two degrees of freedom and creating a permanent coincidence of a focal point of the radiation source and a virtual rotation center located at a distance of the rotation axis. If the virtual rotation center and the focal point coincide, the horizontal and vertical magnification can be kept constant during the scanning process. If the secondary collimator is further placed on the rotary support, a virtual X-ray source is provided that has the same beam characteristics for all angles of deflection so that the geometry of the imaging is considerably simplified.

The first drive unit can be arranged such that the rotary support is pivoted around a virtual rotation center placed at any axial distance between the rotation axis of the rotary support and infinite, so that the apparatus can easily be adapted to different positions of the patient.

In a further embodiment, the detector is moved along a detector trajectory in synchronization with the motion of the intersection of the X-ray beam with the trajectory of the detector, so that a detector with a limited lateral extension can be used.

The primary collimator can be arranged for producing a first roughly shaped X-ray beam, which is further fine collimated by the secondary collimator and which is impinging in an active region of the detector. Thus, the X-ray beam, which impinges on the linear shaped X-ray detector, is generated by the primary and secondary collimator of the rotating unit. The primary collimator and the secondary collimator may have an elongated aperture producing a fan shaped x-ray beam, and the X-ray detector has an elongated active region corresponding to the dimensions of the fan shaped X-ray beam, so that the focal point of the X-ray beam is also the projection center.

Additionally, means may be used for generating a profile of the patient during the scanning process. These means, for example, may include an optical beam slightly offset from the X-ray beam. These means may further include an optical sensor. The profile acquired by these means can be used for modulating operational parameters of the scanning process in dependency of the profile of the patient. For example, the intensity may be increased during the transition from a soft tissue area, such as the tip of the nose, to the bony area of a patient skull.

The operational parameters of the scanning process that can be modulated comprise the intensity of the X-ray beam, the scanning speed and the sensitivity of the X-ray detector. By modulating at least one of these operational parameters, the distribution of the image values of the radiographic image for both soft and hard tissues of the patient can be optimized.

If the modulation is performed automatically in response to a profile of the patient, the workload of the operator can be reduced.

The profile of the patient is reconstructed starting from the acquired profile points in the optical or in the radiographic domain using a statistical method for generating the profile of the patient. With such a method, previous knowledge on typical profiles can be used for determining the profile, especially if the statistical method adapts a parametric model to the acquired profile points and wherein parameters of the models are updated incrementally during the acquisition of the patient profile or in a single step after the acquisition of the entire profile of the patient.

In one embodiment, the profile of the patient is obtained from optical data generated by an optical beam and by an optical sensor anticipating the X-ray beam. Such a beam can effectively be used for determining a silhouette of the patient by detecting the shadowing by the body of the patient. The optical data can be used to reconstruct a profile of the patient in real time. The profile can then be used for modulating the X-ray beam.

In a further embodiment, the modulation can also be performed automatically in real time in response to a feedback signal depending on the X-ray detector dose that corresponds to the actual exposed region. But the feedback signal can also be obtained by the variations of the moving average of the signal level in the exposed area of the X-ray detector.

The modulation may also use a predefined profile of intensity which takes into account the expected variations in the anatomy of the patient. If necessary, the predefined profile can be adjusted in response to the choice of the operator or a sensor input detecting at least one anatomical parameter of the patient.

The method is particularly suited for dental cephalography since the method provides a simplified and economic construction of cephalographies.

An apparatus arranged for performing the method is provided with a secondary collimator mounted on the rotary support. A first driving system for the rotary support further comprises two actuators for linear movement of the rotary support in two different directions.

Further advantages and properties of the present invention are disclosed in the following description, in which exemplary embodiments of the present invention are explained in detail based on the drawings:
- Figure 1: is a view from above on an apparatus for dental cephalography;
- Figure 2: demonstrates the shift of a focal point of the X-ray source due to the linear motion of a rotation axis;
- Figure 3: demonstrates the effect of a non-linear motion of the rotation axis on the stability of the focal point;
- Figure 4: illustrates projections with varying distance between image plane and focal point;
- Figure 5: illustrates projections with varying lateral positions of the focal point;
- Figure 6: is a view on a modified embodiment of the apparatus according to Figure 1 having an optical beam and an optical sensor for determining a silhouette of the patient; and
- Figure 7: is a view from above on a light shielding arrangement in front of the optical sensor.

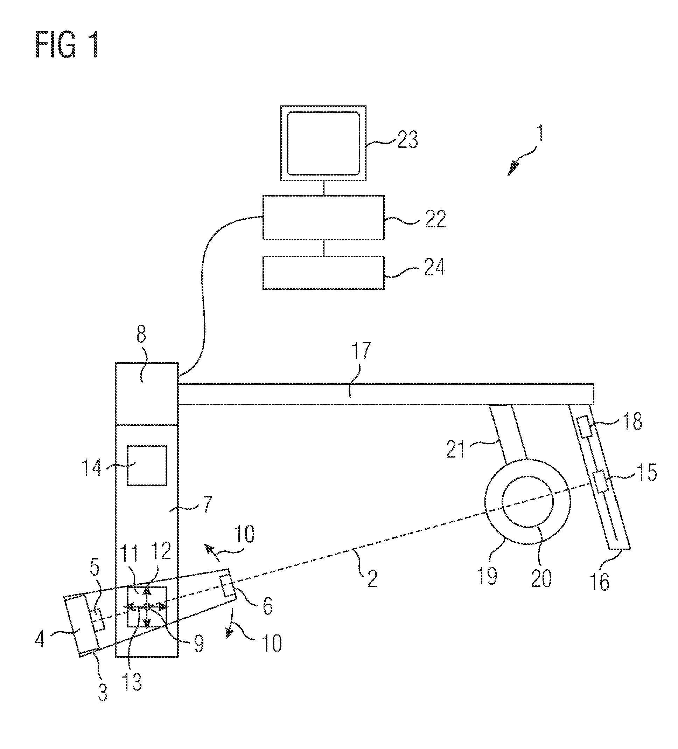

Figure 1 shows a view from above on an apparatus 1 for X-ray scanning, in particular for dental cephalography. The apparatus 1 is preferably a horizontal scanning system, where an X-ray beam 2 is created by a rotary arm 3 accommodating an X-ray source 4, a primary collimator 5 and a secondary collimator 6. The X-ray beam 2 comprises the shape of a fan extending in the vertical direction. The rotary arm 3 is pivotably mounted on a member 7 that is attached to a column 8. The rotary arm 3 can be pivoted in a rotational plane around a rotation axis 9 that defines the rotation center of a rotational movement 10 of the rotary arm 3 within the plane extended by the rotational movement of the rotary arm 3. The rotational motion 10 of the rotary arm 3 is driven by a cinematic unit 11. The cinematic unit 11 also allows for displacing the rotation axis 9 along two horizontal axes 12 and 13. The rotational axis 9 can thus be moved with two degrees of freedom within a plane extending along the rotational plane in which the rotation of the rotary arm 3 occurs. The cinematic unit 11 is preferably provided with multi-axis independent actuators and might be a XY linear table equipped with a rotational actuator.

In most cases, the rotational axis will be vertically aligned so that the rotational plane is a horizontal plane. In consequence also the translational movements of the rotational axis 9 occur in horizontal directions.

The operation of the cinematic unit 11 is controlled by a control unit 14 which is capable of independently driving each axis 12 and 13 of the cinematic unit 11. Thus, a translation of the mechanical rotation center of the rotary arm 3 in the useful range of the XY table and a rotational motion of the whole rotary arm 3 can be performed.

The apparatus 1 further comprises an X-ray detector 15 mounted on a detector arm 16. The X-ray detector 15 may be a CCD sensor operated in TDI mode or a CMOS sensor operated in frame acquisition mode. The detector arm 16 is connected to the column 8 by a connecting strut 17. The X-ray detector 15 is capable of performing a linear translation in a direction extending along the rotational plane, in which the rotation of the rotary support occurs. In the embodiment of Figure 1, the linear translation of the X-ray detector 15 is further synchronized with the motion of the first driving system. The X-ray detector 15 is actuated by a linear drive 18 controlled by the control unit 14. The control unit 14 controls the roto-translation of the rotary arm 3 and the linear translation of the X-ray detector 15. The control unit 14 further controls the synchronization of both motions. Thus, the control unit 14 ensures that the x-ray fan beam 2 always impinges perfectly on the X-ray detector 15 during the scanning process.

During this scanning process, a patient 19, in particular a head 20 of the patient 19 is irradiated by the X-ray beam 2. The head 20 of the patient 19 is held in a stationary position with respect to the apparatus 1 by a positioning device 21, such as a cephalostat. In consequence, the spatial relation between the X-ray source 4, the head 20 and the X-ray detector 15 is known at any time during the scanning process.

The apparatus 1 is further provided with an image processing device 22. The image processing device 22 is arranged for processing the radiographic images generated by the detector 15. In particular, image data generated during the scanning process can be used for generating antero-posterior views or lateral views of the head 20 of the patient 19. These views can be displayed on a display 23 that is connected to the image processing device 22. The image processing device 22 can finally also be connected to an input device 24. The image processing device 22 can be a personal computer, the display device 23 can be a screen and the input device 24 a keyboard, a mouse or a similar device.

The apparatus 1 is operated as follows: After the patient 19 has been positioned with respect to the apparatus 1 by the positioning device 21 the rotary arm 3 is pivoted around the rotation axis 9. While the X-ray beam 3 scans the head 20 of the patient 19 the X-ray detector 15 is read out at a predefined clock frequency, in synchronization with the linear translation of the second driving system. The image data read out form the X-ray detector 15 are then used for reconstructing a radiographic image of the head 20 of the patient 19.

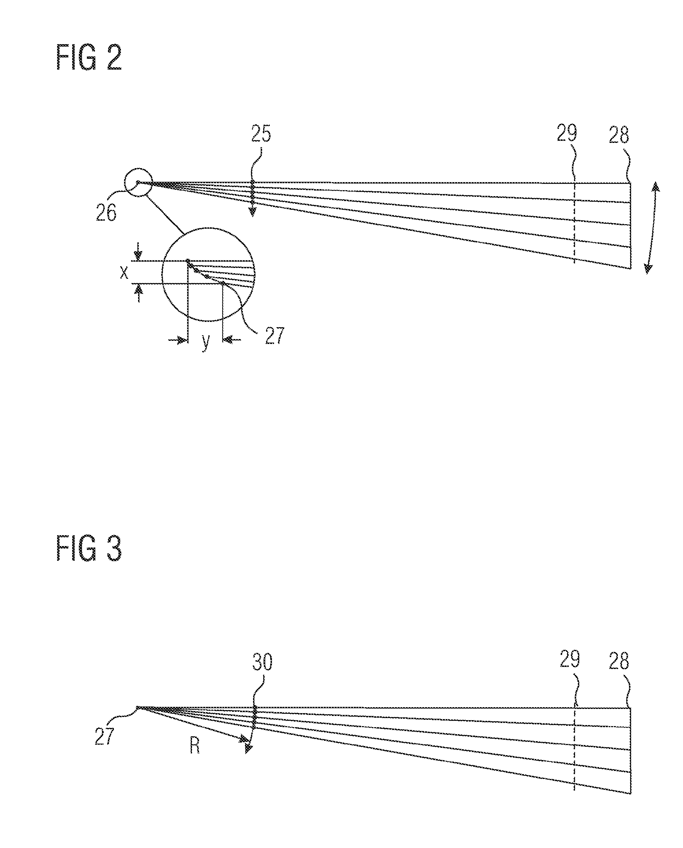

As depicted in Figure 2, the rotation axis 9 can also be displaced during the scanning process. In Figure 2 the rotation axis 9 is displaced by a linear motion 25. The movement of the rotary arm 3 is therefore composed of the rotational motion 10, depicted in Figure 1, around the rotation axis 9 and the linear motion 25. The combined motion of the rotary arm 3 is approximately the same as a rotation around a virtual rotation center 26 that is located at a distance of the actual rotation center defined by the rotation axis 9. Thus, the X-ray beam 2 is effectively pivoted around the virtual rotation center by a deflection angle ϕ. However, the linear motion 25 has also the effect that a focal point 27 of the X-ray source 4 is slightly shifted with respect to the virtual rotation center 26 as can be recognized from the enlarged cut-out within Figure 2. The focal point 27 generally equals with the focal spot on the anode of the X-ray tube forming the X-ray source 4. The position of the focal point also determines the magnification of the apparatus 1 since the horizontal or vertical magnification of the apparatus 1 is defined by the ratio of the Source Image Distance (= SID) to the Source Object Distance (= SOD). The SID is the distance between the focal point 27 and an image plane 28 that is defined by the trajectory of the detector 15 and SOD is the distance between the focal point 27 and an object plane 29 through the object. In the embodiment according to Figure 2, the object plane 29 is a plane that extends along the image plane 28 and intersects the head 20 of the patient 19. As can be recognized from Figure 2 the horizontal and vertical amplification varies due to the shift of the focal point 27 since the shift of the focal point 27 affects also the ratio of SID to SOD.

The focal point 27, however, can be kept in permanent coincidence with the virtual rotation center 26 if the rotation axis 9 is moved in a non-linear way, in particular if the rotation axis 9 is moved along a curved trajectory 30 having the shape of a circular arc as shown in Figure 3. In this case the horizontal and vertical magnifications remain constant and are independent of the angle of deflection ϕ.

The geometry of the imaging is further simplified by disposing the secondary collimator 6 on the rotary arm 3, because the primary collimator 5 and the secondary collimator 6 are always aligned during the scanning process, and because the effective width of the X-ray beam 2 is not reduced at increasing angles of deflection ϕ as in the prior art.

The location of the secondary collimator 6 on the rotary arm together with the stationary focal point 27 creates a virtual X-ray source emitting X-ray beams 2 originating from the same origin and having the same angular width in all directions. Thus, the geometry of the X-ray beam 2 is considerably simplified in comparison with prior art devices.

Another advantage of the arrangement of the secondary collimator 6 is that the arrangement of the secondary collimator 6 on the rotary arm 3 allows for an easy adjustment of the primary collimator 5 and the secondary collimator 6 on the X-ray detector 15, without the need of an accurate synchronization between primary and secondary collimator as in prior art arrangements where the secondary collimator is moved by an independent drive.

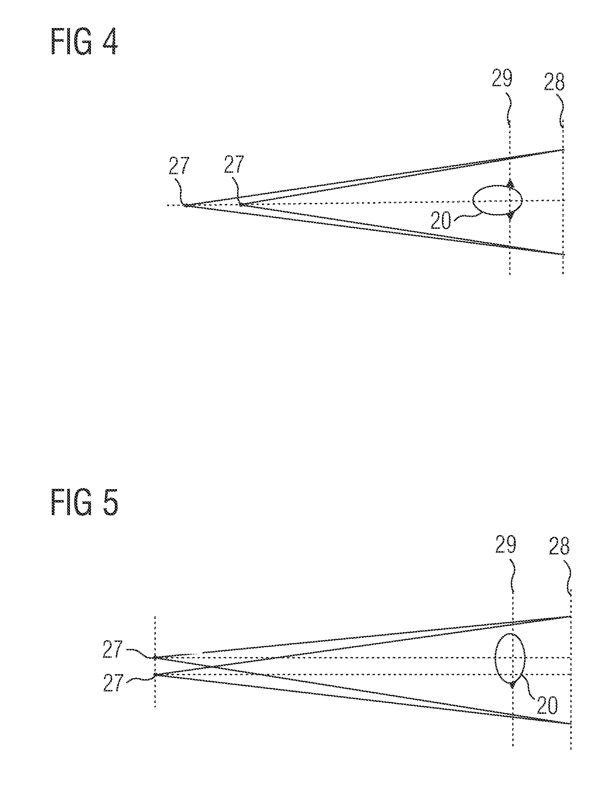

The apparatus 1 is further capable of executing projections with varying SID, as depicted in Figure 4.

The apparatus 1 is also capable of executing projections with varying lateral positioning of the virtual center, as depicted in Figure 5. In these cases, the predefined virtual center is preferably placed at a lateral displacement from the object laying on a mid-sagittal axis for antero-posterior views or laying on an ear meatus axis for lateral views.

For adjusting the virtual rotation center 26 and the focal point 27, the cinematic unit 11 enables a wide range of rototranslations of the rotary arm 3 in a predefined useful range. The predefined range depends on the dimensions of the apparatus 1. If the distance between the X-ray source 4 and the detector 15 ranges between one and two meters, the cinematic unit 11 is arranged for performing a translational motion in the rotational plane in an area of not less than 14 x 14 cm.

For modifying the height of the X-ray fan beam, the primary or secondary collimator aperture may be vertically adjustable.

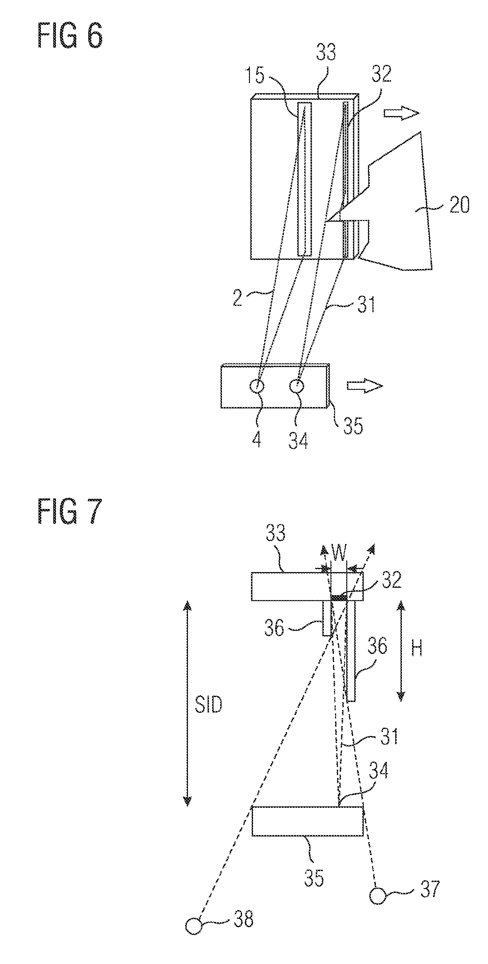

Figure 6 shows an fan shaped optical beam 31 slightly offset from the fan shaped X-ray beam 2, which impinges on an optical sensor 32 consisting of an array of photo-detectors placed beside the detector 15 on the same detector support 33. The optical beam 31 is produced by an optical light source 34 positioned next to the X-ray source 4 on a common support 35. The optical light source 34 can be a coherent light source such as a laser, or an incoherent light source such as a LED. The optical light source 34 is preferably collimated. The area, which is irradiated by the light source on the image plane 28, preferably has the shape of a line and is aligned with optical sensor 32.

While scanning the patient 19, in particular the head 20 of the patient 19, the optical beam 31 slightly anticipates the X-ray beam 2. The optical data acquired by the optical sensor 32 can be used to determine a silhouette of the head 20. Such a silhouette represents a two-dimensional profile which is used to modulate the intensity of the X-ray beam 2 according to a characterizing morphology of the profile of the head 20 such that optimal exposure is achieved; the optical data can also be used to process the cephalometric radiographic image by using knowledge on the position and shape of the profile of the head 20.

As will be explained in detail later on, the profile of the patient can also be reconstructed starting from the acquired profile points in the optical or in the radiographic domain using statistical techniques such as anatomical parametric modeling such as profile parametric models, incremental model fitting or snake adaptive contour detection algorithms.

The optical sensor 32 can extend contiguously over an area whose dimensions are comparable with the dimensions of the active area of the X-ray detector 15. Such an optical sensor 32 allows for acquiring a contiguous profile of the patient 19. For instance, an integrated linear sensor array could be used for such an optical sensor 32.

The optical sensor 32 can also be formed by a set of isolated sensor elements such as individual photodiodes dispersed over the active area. These sensor elements can be uniformly or non-uniformly distributed over the area covered by the optical sensor 32. A non-uniform distribution can, for instance, be used to increase the number of sampled points at those places, where the variability of the profile of the patient is particularly high, for instance in the area of the nose. In this case, the profile of the head 20 has to be interpolated, using a parametric curve or, even better, using a parametric anatomical model.

For the detection of the light from the light source 34 ambient light can be suppressed either by factory calibration, real-time on-site calibration, a lock-in detector, by an appropriate collimation of the optical beam 31, by baffles similar to an anti-scatter grid or by selection of an appropriate wavelength for the optical system.

The wavelength of the optical source 32 can be in the visible range or in another range, in particular the infrared or ultraviolet wavelength range. In this case, the laser wavelength can be chosen such that the detection of the light emitted by the light source 34 is not affected by ambient light from other light sources.

The optical sensor 32 can be calibrated only once, soon after the assembly of the apparatus 1. However, the intensity of the ambient light can vary significantly. For instance, the apparatus 1 could be installed in a room, which is directly illuminated by the sun at some moments and completely obscured at other moments. Therefore, the optical sensor 32 is preferably calibrated just before a scan starts or while scanning in order to limit the variation of the ambient light.

Such a calibration procedure can be performed as follows: When the machine starts moving the light source 34 is initially switched off. Each sensor element of the optical sensor 32 then acquires a predefined number of samples, whose measured mean signal is associated with the typical background level. Then the light source 34 is switched on and the same number of samples is acquired again by each sensor element of the optical sensor 32. Although the scan of the head 20 has already started, the profile of the head 20 has not entered yet into the light path between the light source 34 and the optical sensor 32. The acquired samples are used to determine the typical signal intensity associated with the incidence of light from the light source 34 onto the surfaces of the sensor elements of the optical sensor 32. A threshold is then easily computed for each sensor element, for instance as the mean of the background signal and the light source signal, which allows to distinguish between the presence or absence of light from the light source 34.

It should be noted that the calibration procedure can be applied both to optical sensors 32 of the contiguous type and to optical sensors 32 with dispersed sensor elements.

Besides temporal variation, the optical conditions can also vary spatially inside a room; for instance, light passing through a window can generate some bright areas in a room behind the window. As shown in Figure 7, baffles 36 can effectively suppress light from external light sources 37 and 38. The minimal or maximal angles of incidence of the light ray, that are emitted by the external light source 37 and 38 and that are received by the optical sensor depend on the SID of the apparatus 1, the width W of the optical sensor 32 and the dimensions, for example the width L of the support 35 or of the housing of the X-ray source 4 and the optical source 32. Taking into account the specific geometry, two baffles 36 with height H can be disposed next to the optical sensor 32 so that the ambient light is effectively suppressed.

Similar consideration are valid for light rays incident from above or below. However, it seems less probable that some light sources are positioned in the bottom area of the room. Moreover, the rotary arm 3 can also be an effective shield against light coming from above.

In practice, the optical sensor 32 can be housed in a black box with a small opening, which allows the light from the light source 34 to reach the surface of the optical sensor 32 and to shield the optical sensor 32 against light from other light sources.

Another possibility is to acquire an optical flat field image, with no patient 19 positioned within the apparatus 1.

The flat field image constitutes a background image, which can vary with the position of the optical sensor 32.

The spatial and temporal background light variations can also be suppressed by modulating the light emitted by the light source 34 and by using a lock-in amplifier for detecting the modulated light. For example, at time tₙ the light source 34 is on and the optical sensor 32 acquires a signal that is composed of the background signal and the signal caused by light from the light source 34; at time tₙ₊₁, the light source 34 is switched off, and the optical sensor 32 just acquires a background signal. Only the sensor elements that register a significant signal change are illuminated by the light source 34, while the other sensor elements are shadowed. Since the light source 34 and the optical sensor 32 can work at a high frequency, even fast drifts can be detected.

The measured light signal can be used to drive an Automatic Exposure Control (= AEC) system. For this purpose, the profile of the head 20 has to be determined while the detector 15 and the X-ray source 4 are moving and the radiography is being acquired. In a simple method to localize the profile of the head 20, the number of the sensor elements, which are reached by light from the light source 34 and which are not shadowed by the head 20, is determined. When the number is beyond a predefined threshold, the X-ray beam 2 is modulated such that an adequate exposure of the soft tissue/bone tissue is achieved. Thus, the intensity of the X-ray beam 2, the scanning speed or the sensitivity of the X-ray detector 15 are modulated during the scanning process for optimizing the grey scale of the radiographic picture for both soft and hard tissues of the body of the patient 19. The modulation of the intensity of the X-ray fan beam can be achieved by controlling the tube voltage, the tube current, by controlling the beam attenuation or filtering or by adjusting the beam aperture.

It should be noted that the modulation of the X-ray beam 2 has to take into account the displacement of the optical sensor 32 with respect to the X-ray detector 15. Preferably, the modulation of the X-ray beam 2 is delayed with respect to the actual optical signal generated by the optical sensor 32 so that the X-ray beam is modulated according to the optical signal that is associated with the actual location of the X-ray beam and that has been generated by the optical sensor 32 previously.

For making the modulation of the X-ray beam 2 more reliable a parametric model can be used to describe the profile of the head 20. In particular, an anatomical parametric model, where each parameter has a specific anatomical significance, can be used. The parameters could represent the nose length and inclination, the curvature of the chin, and similar significant features of the anatomy of the head 20. The parametric model must then be fitted to the measured data. In this case, the measured data are generally the points where the transition between the background and the profile occurs.

Various approaches can be used for fitting the model to the acquired optical data. In a first approach, the acquisition of the optical data is completed before the parametric model is fitted to the optical data. This approach requires a large distance between the X-ray detector 15 and the optical sensor 32 so that the optical data can be processed and the profile is recognized before the X-ray beam 2 effectively reaches the head 20. In some cases, the X-ray scan may also start before the model fitting procedure has finished.

In another approach, the parametric model is fitted incrementally to the optical data. For example, each time a new optical datum is measured by identifying a point of the profile of the head 20, the model parameters are updated taking into account the new datum. A typical example of this approach is an incremental least squares fitting algorithm.

When the model parameters are stable, in particular, when the introduction of a new datum does not significantly alter the model parameters, the profile of the head 20 is well fitted. This approach allows the patient profile to be fitted in less time, so that the X-ray modulation can be efficiently performed.

It should be noted that both approaches can be implemented on the basis of optical data supplied by an optical sensor 32 with a contiguous line of sensor elements or an optical sensor 32 with dispersed sensor elements; in the latter case, it is preferred to use a model fitting approach, as the number of sampled point is low and modeling is the only way to achieve a robust, constrained solution.

The obtained optical profile of the patient 19 can also be used for further processing the radiographic image, such as enhancing the visibility of the radiographic profile of the patient 19, optimizing the image contrast, for instance, the contrast of bony versus soft tissue, performing localized image processing, or automatically localizing distinguished anatomical features.

The acquired patient profile can be superimposed onto the acquired radiographic image. For instance, a well visible red profile could be superimposed on the radiographic image. This may help the dentist to individuate some specific anatomical features, for example nose tip, chin tip, especially when the exposure of the soft tissue in the radiographic image is not optimal. The position of the profile can be obtained considering the delay between the optical sensor 32 and the X-ray detector 15 assuming that their acquisition geometry is the same, otherwise some projective transformation should be applied before plotting the patient profile on the radiographic image.

The shape of the profile acquired by the optical sensor 32, or the profile obtained by fitting a parametric model to a reduced number of points can be both refined by the acquired radiographic image. As a matter of fact, the acquired profile can be used as a reliable initial guess for the patient profile, which then is refined using, for instance, a snake algorithm. This procedure tries to find the curve that best fit the data in an image: typically it maximizes the norm of the gradient measured over all the pixels crossed by the curve, and it constrains at the same time some regularity of the curve. For instance, it penalizes curves with high absolute values of the derivatives. The refined profile can be highlighted onto the acquired radiography and used by the dentist to localize some important anatomical feature of the profile of the head 20.

The acquired radiographic image can be subjected to an image enhancement process by post-processing the radiographic image with a contrast enhancement filter. The post processing with a contrast enhancement filter can be performed by utilizing the information of the previously acquired profile of the head 20 obtained by an optical beam.

Dentists are often interested in localizing some important anatomical feature of the profile of the head 20. These features can be highlighted in the acquired radiographic image by enhancing the contrast of the radiographic profile of the head 20, for instance through an Unsharp Masking (= UM) algorithm. Since the rough shape and position of the patient profile are known from the measured patient profile or from the fitted model, the areas of the radiographic profile can be filtered with an UM algorithm. The filtered areas of the radiographic profile can then be mixed with the original image (blending), to get a new radiograph with a contrast enhanced patient profile. Various parameters involved in this procedure, for instance UM parameters, width of the profile area, blending factor and other parameters, can be controlled by the user to get the subjectively best appearance of the processed radiographic image.

The acquired patient profile divides the radiograph in two different areas: an area inside the body and another area outside the body. Different transformation can be applied in these two areas. Typically, the image background can be left unaltered or set to zero. On the other hand, the inner pixel can be processed for enhancing the contrast of the anatomical structures, for instance through gamma transform, global or local histogram equalization or other image processing methods. Preferably, an automatic approach is used to compute the optimal values for linear stretching and gamma transformation. Generally, the approach presented in FROSIO et al. can be extended to find the local optimal values for linear stretching and gamma transform, which highlights at best at a local scale the visibility of the anatomical features.

In particular, the bone contrast enhancement can be profitably coupled through image blending or similar techniques with the localized image treatment to get a radiograph comprising a high bone contrast and optimal visibility of the patient profile.

If properly arranged, the optical beam 31 can also be used for patient positioning. For example, the traditional method used in orthopantomography can also be applied for cephalography. In this method the vertical line generated by the light source 34 has to pass through the basis of the canine tooth. The procedure as such is well known to the skilled person.

In another positioning method, the optical beam 31 is used for producing a profile of the head 20. If the profile of the head 20 is known, the position of a significant anatomical feature can be identified in the patient profile. The positioning device 21 can then be moved with one degree of freedom until the anatomical feature reaches the correct position.

It should be noted that the rotary arm 3 may also be equipped with a second X-ray detector placed in front of the secondary collimator 6. The second detector may be used for dental panoramic radiography. Preferably, the second X-ray detector can be relocated from a first position on the rotary support suitable for panoramic radiography, to a second position at a predefined distance suitable for cephalography.

If such a device is used for panoramic radiography, in which the detector 15 and the optical sensor 32 is moved on an arcuate path around the head 20, the convex hull of the patient profile can be acquired, because the optical beam 31 can be used for identifying these planes in the three-dimensional space that let the light pass through, and those planes in which light is prevented from passing through. On the basis of information on the geometry of the apparatus 1, a volume reconstruction technique can be applied for reconstructing the convex contour of the face of the patient 19. By fitting a parametric model to the convex contour as in VETTER and BLANZ, a three-dimensional model of the head 20 can be determined.

The embodiments described herein are related to a X-ray scanning apparatus 1 of simplified concept, consisting of a rotating unit accommodating X-ray source and primary and secondary collimator, mechanically driven by a powerful multi-axis and wide range cinematic unit 11 capable of performing simultaneous rotation and translation of its mechanical center in the horizontal plane, and a X-ray detector 15 driven by a linear horizontal guide actuator. Thus, an appropriate control unit provides synchronized movements of the rotating unit and detector 15, with full programmability of the cinematic projection from a virtual center located at predefined distance from the detector.

The various embodiments of the apparatus 1 described herein have a number of advantages:
A first advantage is the simplified construction of the horizontal X-ray scanning system, where the secondary collimator is placed on the rotating unit, so that no separate drive is needed for controlling the movement of the secondary collimator. In contrast to the prior art, the radiation source with primary and secondary collimator form a integrated fan shaped X-ray beam generator, whose movements are controlled by a single powerful cinematic unit. This is particularly advantageous in dental panoramic radiography, where a rotating arm driven by a cinematic unit is usually already available.

Another advantage is the availability of a multi-axis wide range cinematic unit to accomplish a variety of scanning projections. For example, by simply changing the software settings or by user selection, it is possible to perform parallel scanning, as well as scanning from a virtual rotation center placed at any predefined SID.

It also advantageous to avoid a possible movements of the patient, usually held in place by the help of a patient support system, and to utilize the roto-translation capabilities of the cinematic unit and the associated control unit to acquire various kinds of views of the patient, for instance partialized views, extended views, anterior-posterior views, lateral views and similar views with a high degree of flexibility regarding the horizontal positioning of the virtual center of the projection.

By the optical fan beam a patient silhouette can be determined which allows for modulating the intensity of the X-ray fan beam in order to compensate the variance of the tissues X-ray absorption, thus leading to a stable signal regarding soft tissue areas and bony tissue areas.

It is further an advantage to have a scanning system where the effective aperture of the secondary collimator 6, hence the width of the X-ray beam 2, is constant along the whole scanning range without reduction of the aperture at increasing angle of deflection.

Throughout the description and claims of this specification, the singular encompasses the plural unless the context otherwise requires. In particular, where the indefinite article is used, the specification is to be understood as contemplating plurality as well as singularity, unless the context requires otherwise.

## Claims

1. A method for digital x-ray scanning for dental cephalography, the method comprising the steps of:
- positioning a patient (19) with respect to a radiation source (4) and a detector (15) using a patient support system (21) ;
- generating X-ray radiation (2) by the radiation source (4) ;
- performing a scan of the patient (19) by performing a roto-translation of a rotary support (3) supporting the radiation source (4) and a primary collimator (5) disposed between the radiation source (4) and the patient support system (21);
- moving a secondary collimator (6) in synchronization with the rotary support (3);
- detecting the X-ray radiation (2) by means of the detector (15);
- performing a readout of the detector (15) for generating a radiographic image of the patient (19);
**characterized in that**
- the secondary collimator (6) is mounted on the rotary support (3) together with the radiation source and primary collimator and that, for scanning the patient for dental cephalography, the secondary collimator (6) is moved together with the radiation source (4) and the primary collimator (5) by moving the rotary support (3).

2. The method according to Claim 1, wherein the roto-translation during scanning is composed of a rotational motion (10) around a rotation axis (9) and a displacement (11, 12) of the rotation axis (9) comprising two degrees of freedom and keeping a focal point (27) of the radiation source (4) in permanent coincidence with a virtual rotation center (26) located at a distance of the rotation axis (9).

3. The method according to Claim 2,
wherein the rotary support (3) is pivoted around a virtual rotation center (26) placed at any axial distance between the rotation axis (9) of the rotary support (3) and infinite.

4. The method according to any one of Claims 1 to 3,
wherein the detector (15) is moved along a detector trajectory in synchronization with the motion of the intersection of the X-ray beam (2) with the trajectory of the detector (15).

5. The method according to any one of Claims 1 to 4,
wherein the primary collimator (5) is arranged for producing a first roughly shaped X-ray beam (2), which is further fine collimated by the secondary collimator (6) and which is impinging in an active region of the detector (15).

6. The method according to Claim 5,
wherein the primary collimator (5) and the secondary collimator (6) have an elongated aperture producing a fan shaped X-ray beam (2), and the detector (15) has an elongated active region corresponding to the dimensions of the fan shaped X-ray beam (2).

7. The method according to any one of Claims 1 to 6,
wherein at least one of the operational parameters of the scanning process comprising the intensity of the X-ray fan beam (2), the scanning speed and the sensitivity of the detector (15) is modulated during the scanning process for optimizing the distribution of the image values of the radiographic image for both soft and hard tissues of the patient (19).

8. The method according to Claim 7,
wherein the modulation is performed automatically in response to a profile of the patient (19).

9. The method according to Claim 8,
wherein the profile of the patient (19) is reconstructed starting from optically or radiographically acquired profile points using a statistical method for generating the silhouette of the patient (19).

10. The method according to Claim 9,
wherein the statistical method adapts a parametric model to the acquired profile points and wherein parameters of the models are updated incrementally during the acquisition of the profile of the patient (19) or in a single step after the acquisition of the entire profile of the patient (19).

11. The method according to any one of Claims 8 to 10,
wherein the profile of the patient (19) is obtained from optical data generated by an optical beam (31) and by an optical sensor (32) anticipating the X-ray beam (2).

12. The method according to any one of Claims 7 to 11,
wherein the modulation is performed automatically in real time in response to a feedback signal depending on the detector dose that corresponds to the actual exposed region.

13. The method according to Claim 12,
wherein the feedback signal is obtained by the variations of the moving average of the signal level in the exposed area of the detector (15).

14. The method according to any one of Claims 7 to 13,
wherein the modulation is performed according to a predefined profile of intensity which takes into account the expected variations in the anatomy of the patient (19).

15. The method according to any one of Claims 7 to 14,
wherein the predefined profile is adjusted in response to the choice of an operator or in response to a sensor input detecting at least one anatomical parameter of the patient (19).

16. An apparatus for digital X-ray scanning for dental cephalography, comprising:
- a radiation source (4) arranged for generating X-ray radiation (2),
- a detector (15) arranged for detecting said X-ray radiation (2),
- a patient support system (21) for positioning a patient (19) in a radiation path between the radiation source (4) and the detector (15);
- a primary collimator (5) positioned in the radiation path between the radiation source (4) and the patient support system (21);
- a secondary collimator (6) disposed in the radiation path between the primary collimator (5) and the patient support system (21);
- a rotary support (3) on which the radiation source (4) and the primary collimator (5) are mounted;
- a driving system (11) capable of performing a roto-translation of the rotary support (3) for moving the radiation source (4) and the primary collimator (5) during a scanning process, wherein the driving system (11) comprises two actuators for linear movement of the rotary support (3) in two different directions;
- a control unit (14) for controlling the driving system and the data acquisition during the scanning process; and
- an imaging processing device (22) for generating radiographic images,
**characterized in that**
- the secondary collimator (6) is mounted on the rotary support (3), and that
- the driving system (11), the control unit (14) and the image processing device (22) are arranged for performing a method according to any one of claims 1 to 15.

## Patentansprüche

1. Verfahren für digitales Röntgenstrahlen-Scannen für die dentale Cephalographie,
wobei das Verfahren die Verfahrensschritte umfasst:
- Positionieren eines Patienten (19) bezüglich einer Strahlungsquelle (4) und eines Detektors (15) mit Hilfe einer Patientenhaltevorrichtung (21);
- Erzeugen von Röntgenstrahlung (2) durch die Strahlungsquelle (4);
- Ausführen eines Scans des Patienten (19) durch Ausführen einer Drehverschiebung einer Drehhalterung (3), die die Strahlungsquelle (4) und einen primären Kollimator (5) hält, der zwischen der Strahlungsquelle (4) und der Patientenhaltevorrichtung (21) angeordnet ist;
- Bewegen eines sekundären Kollimators (6) synchron mit der Drehhalterung (3);
- Erfassen der Röntgenstrahlung (2) mit Hilfe des Detektors (15) ;
- Ausführen eines Auslesevorgangs des Detektors (15), um ein radiographisches Bild des Patienten (19) zu erzeugen;
**dadurch gekennzeichnet, dass**
- der sekundäre Kollimator (6) an der Drehhalterung (3) zusammen mit der Strahlungsquelle und dem primären Kollimator angebracht ist und dass zum Scannen des Patienten für die dentale Cephalographie der sekundäre Kollimator (6) zusammen mit der Strahlungsquelle (4) und dem primären Kollimator (5) durch Bewegen der Drehhalterung (3) bewegt wird.

2. Verfahren nach Anspruch 1,
bei dem die Drehverschiebung während des Scannens aus einer Drehbewegung (10) um eine Drehachse (9) und einer Verschiebung (11, 12) der Drehachse (9) zusammengesetzt ist, die zwei Freiheitsgrade aufweist und einen Brennpunkt (27) der Strahlungsquelle (4) in dauerhafter Übereinstimmung mit einem virtuellen Drehzentrum (26) hält, das sich in einem Abstand zur Drehachse (9) befindet.

3. Verfahren nach Anspruch 2,
bei dem die Drehhalterung (3) um ein virtuelles Drehzentrum (26) verschwenkt wird, das in einer beliebigen axialen Entfernung zwischen der Drehachse (9) der Drehhalterung (3) und Unendlich angeordnet ist.

4. Verfahren nach einem der Ansprüche 1 bis 3,
bei dem der Detektor (15) entlang einer Detektor-Trajektorie synchron mit der Bewegung eines Schnittpunkts des Röntgenstrahls (2) mit der Trajektorie des Detektors (15) bewegt wird.

5. Verfahren nach einem der Ansprüche 1 bis 4,
bei dem der primäre Kollimator (5) dazu eingerichtet ist, einen ersten, grob geformten Röntgenstrahl (2) zu erzeugen, der durch den zweiten Kollimator (6) weiter feinkollimiert wird und der auf einen aktiven Bereich des Detektors (15) trifft.

6. Verfahren nach Anspruch 5,
bei dem der primäre Kollimator (5) und der sekundäre Kollimator (6) eine langgestreckte Apertur aufweisen, die einen fächerförmigen Röntgenstrahl (2) erzeugt, und bei dem der Detektor (15) einen langgestreckten aktiven Bereich aufweist, der den Abmessungen des fächerförmigen Röntgenstrahls (2) entspricht.

7. Verfahren nach einem der Ansprüche 1 bis 6,
bei dem wenigstens einer der Betriebsparameter des Scanvorgangs, die die Intensität des fächerförmigen Röntgenstrahls (2), die Scangeschwindigkeit und die Empfindlichkeit des Detektors (15) umfassen, während des Scanvorgangs moduliert wird, um die Verteilung der Bildwerte des radiographischen Bildes sowohl für weiches als auch hartes Gewebe des Patienten (19) zu optimieren.

8. Verfahren nach Anspruch 7,
bei dem die Modulation automatisch in Reaktion auf ein Profil des Patienten (19) ausgeführt wird.

9. Verfahren nach Anspruch 8,
bei dem das Profil des Patienten (19) ausgehend von optisch oder radiographisch aufgenommenen Profilpunkten erstellt wird, indem ein statistisches Verfahren zum Erzeugen des Umrisses des Patienten (19) verwendet wird.

10. Verfahren nach Anspruch 9,
bei dem das statistische Verfahren ein Parametermodell an die aufgenommenen Profilpunkte anpasst und bei dem Parameter des Modells inkrementell während der Aufnahme des Profils des Patienten (19) oder in einem einzigen Verfahrensschritt nach der Aufnahme des gesamten Profils des Patienten (19) aktualisiert werden.

11. Verfahren nach einem der Ansprüche 8 bis 10,
bei dem das Profil des Patienten (19) aus optischen Daten erhalten wird, die von einem optischen Strahl (31) und einem optischen Sensor (32) erzeugt werden, die den Röntgenstrahl (2) vorwegnehmen.

12. Verfahren nach einem der Ansprüche 7 bis 11,
bei dem die Modulation automatisch in Echtzeit in Reaktion auf ein Rückmeldesignal ausgeführt wird, das von der Detektordosis abhängt, die dem tatsächlich bestrahlten Bereich entspricht.

13. Verfahren nach Anspruch 12,
bei dem das Rückmeldesignal durch die Änderungen des gleitenden Durchschnitts der Signalstärken in dem bestrahlten Bereich des Detektors (15) erhalten wird.

14. Verfahren nach einem der Ansprüche 7 bis 13,
bei dem die Modulation gemäß einem vorbestimmten Intensitätsprofil ausgeführt wird, das die erwarteten Abweichungen in der Anatomie des Patienten (19) berücksichtigt.

15. Verfahren nach einem der Ansprüche 7 bis 14,
bei dem das vorbestimmte Profil in Reaktion auf die Wahl eines Bedieners oder in Reaktion auf einer Sensoreingangsgröße eingestellt wird, die wenigstens einen anatomischen Parameter des Patienten (19) erfasst.

16. Vorrichtung für digitales Röntgenstrahlen-Scannen für die dentale Cephalographie mit:
- einer Strahlungsquelle (4), die zum Erzeugen von Röntgenstrahlung (2) eingerichtet ist;
- einem Detektor (15), der zum Erfassen dieser Röntgenstrahlung (2) eingerichtet ist;
- einer Patientenhaltevorrichtung (21) zum Positionieren eines Patienten (19) im Strahlengang zwischen der Strahlungsquelle (4) und dem Detektor (15);
- einem primären Kollimator (5), der im Strahlengang zwischen der Strahlungsquelle (4) und der Patientenhaltevorrichtung (21) angeordnet ist;
- einem sekundären Kollimator (6), der im Strahlengang zwischen dem ersten Kollimator (5) und der Patientenhaltevorrichtung (21) angeordnet ist;
- einer Drehhalterung (3), an der die Strahlungsquelle (4) und der primäre Kollimator (5) angebracht sind;
- einem Antriebssystem (11), das in der Lage ist, eine Drehverschiebung der Drehhalterung (3) auszuführen, um die Strahlungsquelle (4) und den primären Kollimator (5) während eines Scanvorgangs zu bewegen, wobei das Antriebssystem (11) zwei Stellantriebe für die lineare Bewegung der Drehhalterung (3) in zwei verschiedene Richtungen aufweist;
- einer Steuereinheit (14) zum Steuern des Antriebssystems und der Datenerfassung während des Scanvorgangs; und
- einer Bildverarbeitungsvorrichtung (22) zum Erzeugen von radiographischen Bildern,
**dadurch gekennzeichnet, dass**
- der sekundäre Kollimator (6) an der Drehhalterung (3) angebracht ist und dass
- das Antriebssystem (11), die Steuereinheit (14) und die Bildverarbeitungsvorrichtung (22) dazu eingerichtet sind, ein Verfahren nach einem der Ansprüche 1 bis 15 auszuführen.

## Revendications

1. Un procédé de balayage numérique à rayons X destiné à une céphalographie dentaire,
le procédé comprenant les opérations suivantes :
- le positionnement d'un patient (19) par rapport à une source de rayonnement (4) et un détecteur (15) au moyen d'un système de support de patient (21),
- la génération d'un rayonnement de rayons X (2) par la source de rayonnement (4),
- l'exécution d'un balayage du patient (19) par l'exécution d'une rototranslation d'un support rotatif (3) soutenant la source de rayonnement (4) et d'un collimateur primaire (5) disposé entre la source de rayonnement (4) et le système de support de patient (21),
- le déplacement d'un collimateur secondaire (6) en synchronie avec le support rotatif (3),
- la détection du rayonnement de rayons X (2) au moyen du détecteur (15),
- l'exécution d'un relevé du détecteur (15) de façon à générer une image radiographique du patient (19),
**caractérisé en ce que**
- le collimateur secondaire (6) est monté sur le support rotatif (3) conjointement avec la source de rayonnement et le collimateur primaire et **en ce que**, de façon à balayer le patient en vue d'une céphalographie, le collimateur secondaire (6) est déplacé conjointement avec la source de rayonnement (4) et le collimateur primaire (5) par le déplacement du support rotatif (3).

2. Le procédé selon la Revendication 1,
dans lequel la roto-translation au cours du balayage se compose d'un mouvement de rotation (10) autour d'un axe de rotation (9) et d'un déplacement (11, 12) de l'axe de rotation (9) comprenant deux degrés de liberté et le maintien d'un point focal (27) de la source de rayonnement (4) en coïncidence permanente avec un centre de rotation virtuel (26) situé à une distance de l'axe de rotation (9).

3. Le procédé selon la Revendication 2,
dans lequel le support rotatif (3) est pivoté autour d'un centre de rotation virtuel (26) placé au niveau d'une distance axiale quelconque entre l'axe de rotation (9) du support rotatif (3) et l'infini.

4. Le procédé selon l'une quelconque des Revendications 1 à 3,
dans lequel le détecteur (15) est déplacé le long d'une trajectoire de détecteur en synchronie avec le mouvement de l'intersection du faisceau de rayons X (2) avec la trajectoire du détecteur (15).

5. Le procédé selon l'une quelconque des Revendications 1 à 4,
dans lequel le collimateur primaire (5) est agencé de façon à produire un premier faisceau de rayons X de forme grossière (2), qui est ensuite collimaté finement par le collimateur secondaire (6) et qui empiète dans une zone active du détecteur (15).

6. Le procédé selon la Revendication 5,
dans lequel le collimateur primaire (5) et le collimateur secondaire (6) possèdent une ouverture allongée produisant un faisceau de rayons X en forme d'éventail (2), et le détecteur (15) possède une zone active allongée correspondant aux dimensions du faisceau de rayons X en forme d'éventail (2).

7. Le procédé selon l'une quelconque des Revendications 1 à 6,
dans lequel au moins un des paramètres opérationnels du procédé de balayage comprenant l'intensité du faisceau en éventail de rayons X (2), la vitesse de balayage et la sensibilité du détecteur (15) est modulé au cours du procédé de balayage de façon à optimiser la distribution des valeurs d'image de l'image radiographique pour à la fois des tissus durs et mous du patient (19).

8. Le procédé selon la Revendication 7,
dans lequel la modulation est exécutée automatiquement en réponse à un profil du patient (19).

9. Le procédé selon la Revendication 8,
dans lequel le profil du patient (19) est reconstruit à partir de points de profil acquis optiquement ou par radiographie au moyen d'un procédé statistique destiné à la génération de la silhouette du patient (19).

10. Le procédé selon la Revendication 9,
dans lequel le procédé statistique adapte un modèle paramétrique aux points de profil acquis et dans lequel des paramètres des modèles sont actualisés de manière incrémentale au cours de l'acquisition du profil du patient (19) ou en une opération unique après l'acquisition de la totalité du profil du patient (19).

11. Le procédé selon l'une quelconque des Revendications 8 à 10,
dans lequel le profil du patient (19) est obtenu à partir de données optiques générées par un faisceau optique (31) et par un capteur optique (32) anticipant le faisceau de rayons X (2).

12. Le procédé selon l'une quelconque des Revendications 7 à 11,
dans lequel la modulation est exécutée automatiquement en temps réel en réponse à un signal de rétroaction qui dépend de la dose de détecteur qui correspond à la zone effectivement exposée.

13. Le procédé selon la Revendication 12,
dans lequel le signal de rétroaction est obtenu par les variations de la moyenne de déplacement du niveau de signal dans la zone exposée du détecteur (15).

14. Le procédé selon l'une quelconque des Revendications 7 à 13,
dans lequel la modulation est exécutée selon un profil d'intensité prédéfini qui prend en compte les variations attendues dans l'anatomie du patient (19).

15. Le procédé selon l'une quelconque des Revendications 7 à 14, dans lequel le profil prédéfini est ajusté en réponse au choix d'un opérateur ou en réponse à une entrée de capteur détectant au moins un paramètre anatomique du patient (19).

16. Un appareil de balayage numérique à rayons X destiné à une céphalographie dentaire, comprenant :
- une source de rayonnement (4) agencée de façon à générer un rayonnement de rayons X (2),
- un détecteur (15) agencé de façon à détecter ledit rayonnement de rayons X (2),
- un système de support de patient (21) destiné au positionnement d'un patient (19) dans un trajet de rayonnement entre la source de rayonnement (4) et le détecteur (15),
- un collimateur primaire (5) positionné dans le trajet de rayonnement entre la source de rayonnement (4) et le système de support de patient (21),
- un collimateur secondaire (6) disposé dans le trajet de rayonnement entre le collimateur primaire (5) et le système de support de patient (21),
- un support rotatif (3) sur lequel la source de rayonnement (4) et le collimateur primaire (5) sont montés,
- un système d'entraînement (11) capable d'exécuter une roto-translation du support rotatif (3) destinée au déplacement de la source de rayonnement (4) et du collimateur primaire (5) au cours d'un processus de balayage, le système d'entraînement (11) comprenant deux actionneurs destinés à un déplacement linéaire du support rotatif (3) dans deux directions différentes,
- une unité de commande (14) destinée à la commande du système d'entraînement et des données acquisition au cours du procédé de balayage, et
- un dispositif de traitement d'images (22) destiné à la génération d'images radiographiques,
**caractérisé en ce que**
- le collimateur secondaire (6) est monté sur le support rotatif (3), et **en ce que**
- le système d'entraînement (11), l'unité de commande (14) et dispositif de traitement d'images (22) sont agencés de façon à exécuter un procédé selon l'une quelconque des Revendications 1 à 15.
